# EUROPEAN PATENT APPLICATION

(11) **EP 4 744 707 A1**
(43) Date of publication of application: **20.05.2026**
(21) Application number: 25168179.7
(22) Date of filing: 03.04.2025
(51) Int. Cl.: A61M 21/00, H04R 5/033, H04R 5/04

(54) **SOUND ADJUSTMENT DEVICE AND SOUND SUPPLY SYSTEM**

(30) Priority: 18.11.2024 TW 113144268
(71) Applicant: Cotron Corporation, Taipei City 111 (TW)
(72) Inventor: YANG, Bill, Taipei City 111 (TW)
(74) Representative: Becker, Eberhard

(57) **Abstract**

A sound adjustment device (100, 102, 104) and a sound supply system (50, 60) are provided. The sound adjustment device (100, 102, 104) includes an audio signal receiving element (110), a frequency adjuster (130), and an output port (140). The audio signal receiving element (110) is used to receive an audio signal. The frequency adjuster (130) is signally connected to the audio signal receiving element (110), and is used to adjust the audio signal into a left ear audio signal and a right ear audio signal. A first frequency of the left ear audio signal and a second frequency of the right ear audio signal have a frequency difference. The frequency difference is determined by the frequency adjuster (130). The output port (140) is connected to an earphone (10), and is used to output the left ear audio signal to a left ear speaker (12) of the earphone (10) and output the right ear audio signal to a right ear speaker (14) of the earphone (10).

## Description

### BACKGROUND

### Technical Field

The disclosure relates to an adjustment device and a supply system, and in particular to a sound adjustment device and a sound supply system.

### Description of Related Art

With the rapid advancement of current technology and the flourishing development of audio-visual content, people are increasingly using earphones to listen to music or enjoy audio-visual content while avoiding disturbing others around them. However, how to enhance the user experience of earphones is an area that earphone manufacturers are continuously improving.

### SUMMARY

The disclosure provides a sound adjustment device and a sound supply system, which enable a user to experience binaural beats.

A sound adjustment device of the disclosure includes an audio signal receiving element, a frequency adjuster, and an output port. The audio signal receiving element is used to receive an audio signal. The frequency adjuster is signally connected to the audio signal receiving element and is used to adjust the audio signal into a left ear audio signal and a right ear audio signal. A first frequency of the left ear audio signal and a second frequency of the right ear audio signal have a frequency difference. The frequency difference is determined by the frequency adjuster. The output port is used to connect to an earphone, output the left ear audio signal to a left ear speaker of the earphone, and output the right ear audio signal to a right ear speaker of the earphone.

A sound supply system of the disclosure includes a physiological information sensor, a processor, and a sound adjustment device. The physiological information sensor is used to sense physiological information of a user. The processor is signally connected to the physiological information sensor to receive the physiological information and generates physiological state information according to the physiological information. The sound adjustment device includes an audio signal receiving element, a physiological state information receiving element, a frequency adjuster, and an output port. The audio signal receiving element is used to receive an audio signal. The physiological state information receiving element is used to receive the physiological state information. The frequency adjuster is signally connected to the audio signal receiving element and the physiological state information receiving element and is used to adjust the audio signal into a left ear audio signal and a right ear audio signal. A first frequency of the left ear audio signal and a second frequency of the right ear audio signal have a frequency difference. The frequency difference is determined by the frequency adjuster according to the physiological state information. The output port is used to output the left ear audio signal to a left ear speaker and output the right ear audio signal to a right ear speaker.

In an embodiment of the disclosure, the frequency difference automatically cycles and changes over time.

In an embodiment of the disclosure, the physiological state information indicates whether the physiological state of the user is awake, about to fall asleep, or sleeping.

In an embodiment of the disclosure, the sound adjustment device further includes an audio source, which is signally connected to the audio signal receiving element and is used to provide the audio signal.

In an embodiment of the disclosure, the audio source stores the audio signal or generates the audio signal through a program.

In an embodiment of the disclosure, the processor is separate from the sound adjustment device, and the physiological state information receiving element wirelessly receives the physiological state information.

In an embodiment of the disclosure, the processor is built into the sound adjustment device.

In an embodiment of the disclosure, the sound adjustment device is a mobile phone.

In an embodiment of the disclosure, the output port is a wired connector or a wireless signal transmitter.

Based on the above, in the sound adjustment device and the sound supply system of the disclosure, the corresponding effects beyond hearing sound waves may be generated for users according to their physiological state.

### BRIEF DESCRIPTION OF THE DRAWINGS

FIG. 1 is a circuit block diagram illustrating a sound supply system and a sound adjustment device thereof according to an embodiment of the disclosure.
FIG. 2 is a circuit block diagram illustrating another embodiment of the sound supply system and the sound adjustment device thereof according to the disclosure.
FIG. 3 is a circuit block diagram illustrating yet another embodiment of the sound adjustment device according to the disclosure.

### DESCRIPTION OF THE EMBODIMENTS

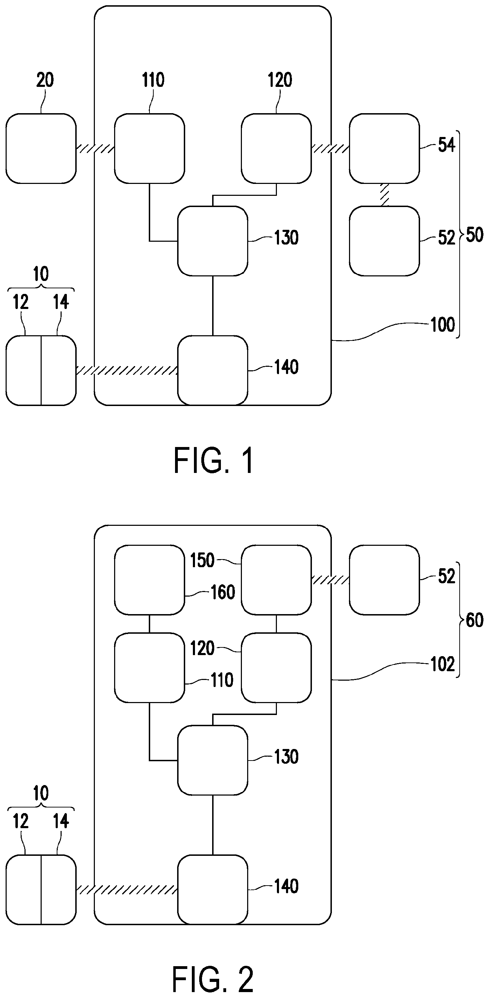

FIG. 1 is a circuit block diagram illustrating a sound supply system and a sound adjustment device thereof according to an embodiment of the disclosure. Referring to FIG. 1, a sound supply system 50 of this embodiment includes a physiological information sensor 52, a processor 54, and a sound adjustment device 100 according to an embodiment of the disclosure. The physiological information sensor 52 is used to sense physiological information of a user. The physiological information measured by the physiological information sensor 52 may include one or more types of physiological information, such as heartbeat, blood pressure, perspiration level, or video images. The physiological information sensor 52 may be regularly worn on the user's body or may be a specialized medical device.

The processor 54 is signally connected to the physiological information sensor 52 to receive the physiological information and generates physiological state information based on the physiological information. The physiological state information, for example, indicates whether the physiological state of the user is awake, about to fall asleep, or sleeping, but the disclosure is not limited thereto.

The sound adjustment device 100 of this embodiment includes an audio signal receiving element 110, a physiological state information receiving element 120, a frequency adjuster 130, and an output port 140. The audio signal receiving element 110 is used to receive an audio signal. The physiological state information receiving element 120 is used to receive the physiological state information. The frequency adjuster 130 is signally connected to the audio signal receiving element 110 and the physiological state information receiving element 120 and is used to adjust the audio signal into a left ear audio signal and a right ear audio signal. A first frequency of the left ear audio signal and a second frequency of the right ear audio signal have a frequency difference. The frequency difference is determined by the frequency adjuster 130 based on the physiological state information. That is, depending on the received physiological state information, the frequency adjuster 130 may adopt different methods to adjust the audio signal and make the frequency difference between the first frequency of the left ear audio signal and the second frequency of the right ear audio signal different.

The output port 140 is used to connect to an earphone 10, output the left ear audio signal to a left ear speaker 12 of the earphone 10, and output the right ear audio signal to a right ear speaker 14 of the earphone 10. The left ear speaker 12 and the right ear speaker 14 respectively transmit the left ear audio signal and the right ear audio signal to the user in the form of sound waves. In this way, the frequency difference mentioned above exists between the sound waves received by the user's left ear and right ear, and the user experiences different sensations due to the varying frequency difference. The output port 140 may be a wired connector such as a 3.5φ audio connector or a USB Type-C connector, or a wireless signal transmitter such as Bluetooth.

In the sound adjustment device and sound supply system of this embodiment, the frequency adjuster determines the frequency difference based on the physiological state information, enabling the user to achieve corresponding effects while listening to the sound waves according to their physiological state.

Brainwaves refer to the electrical oscillations generated by the activity of nerve cells in the human brain, which may also be described as the rhythm of brain cell activity. Regardless of the time, the human brain continuously generates brainwaves. Based on frequency classification, brainwaves at least include β waves, α waves, θ waves, δ waves, and γ waves. Generally, when a person is in the third stage of non-rapid eye movement sleep, the brainwaves are typically δ waves (frequencies between 0.5 Hz and 4 Hz). When a person is in states such as deep dreaming, deep meditation, or subjective intensity, the brainwaves are typically θ waves (frequencies between 4 Hz and 8 Hz). When a person is in states such as pre-sleep drowsiness, gradual loss of consciousness, sudden inspiration, or a relaxed and focused state, the brainwaves are typically α waves. When a person is in states such as relaxation but mental focus or processing previously received information, the brainwaves are typically β waves. Research has also shown that when the brain is induced to produce specific brainwaves, it can, in turn, influence a person's state. For example, when the brain is induced to generate α waves, the person may be brought into a state of sudden inspiration, relaxed body and mind, and focused attention.

By adjusting the frequency of the audio signal through the frequency adjuster 130, the frequency difference between the first frequency of the left ear audio signal and the second frequency of the right ear audio signal may be set. This allows the user's brain to be induced to generate β waves, α waves, θ waves, δ waves, γ waves, or other brainwaves, leading the user into the corresponding states mentioned above, such as enhancing focus, improving sleep quality, relieving stress, or entering meditation.

In this embodiment, the frequency difference may automatically cycle and change over time. In other words, the frequency difference is not fixed and unchanging. Moreover, no manual intervention is required, as the frequency difference will automatically change according to a preset method. Additionally, the method of changing the frequency difference may be preconfigured. Alternatively, there may be multiple methods of variation, and the user may choose which variation method to use. Furthermore, before deciding on the method for changing the frequency difference, the physiological state of the user may be detected to determine the optimal variation method, reflecting differences in the user's physiological state. However, an interface may also be provided for the user to make adjustments to the method of changing the frequency difference. For example, the sound adjustment device 100 may be a mobile phone, which may optionally provide a corresponding application program. Through the interface provided by the application program, the user may adjust the frequency difference or the method of changing the frequency difference. Of course, the user may also allow the sound adjustment device 100 to automatically adjust the audio signal in different ways based on the received physiological state information using a preset method.

For example, the frequency difference may automatically and incrementally cycle between 8 Hz and 12 Hz, with each change in frequency difference being 0.1 Hz and maintaining the same frequency difference for 10 seconds after each change. The automatic and incremental cycling of the frequency difference between 8 Hz and 12 Hz means, for example, that the frequency difference gradually changes from 8 Hz to 12 Hz and then gradually changes from 12 Hz to 8 Hz, or that it repeatedly and gradually changes from 8 Hz to 12 Hz, or repeatedly and gradually changes from 12 Hz to 8 Hz.

In this embodiment, the frequency difference may automatically cycle between 0.5 Hz and 4 Hz to induce δ waves. The frequency difference may automatically cycle between 4 Hz and 8 Hz to induce θ waves. The frequency difference may automatically cycle between 8 Hz and 12 Hz to induce α waves. The frequency difference may automatically cycle between 12 Hz and 20 Hz to induce β waves. The frequency difference may automatically cycle between 25 Hz and 40 Hz to induce γ waves. Additionally, in this embodiment, if the time period is too short, the frequency difference may only gradually increase or only gradually decrease.

Each user's brain does not have a single frequency difference that produces the best induction effect. Typically, multiple frequency differences may produce optimal induction effects. Since the sound adjustment device in this embodiment induces the user with an automatically cycling frequency difference, it may achieve better induction effects across multiple frequency differences. Although a single input frequency difference may produce an optimal induction effect for a test subject, the duration of this effect may not be long. Because the sound adjustment device in this embodiment induces the user with an automatically cycling frequency difference within a selected range, it may switch to another frequency difference after the user's brain becomes fatigued, thereby achieving another optimal induction effect. Ultimately, the sound adjustment device in this embodiment may produce better brainwave induction effects over an extended period of time.

In this embodiment, the processor 54 is separate from the sound adjustment device 100, and the physiological state information receiving element 120 wirelessly receives the physiological state information. For example, the processor 54 may be a cloud-based device that wirelessly or via a wired network receives physiological information from the physiological information sensor 52. The processor 54 determines the physiological state information based on the physiological information in a preset manner and wirelessly transmits the physiological state information to the physiological state information receiving element 120. Optionally, when the processor 54 determines the physiological state information, it may involve professional assistance to verify the physiological state information and, if necessary, adjust the physiological state information transmitted to the physiological state information receiving element 120.

In this embodiment, the audio signal receiving element 110 may receive audio signals wirelessly or via a wired network from an external audio source 20. The audio signal may be stereo or mono, and the disclosure does not impose limitations on this. The left ear speaker 12 and the right ear speaker 14 may be part of a pair of earphones, such as dynamic speakers, balanced armature speakers, bone conduction speakers, or other types of speakers.

FIG. 2 is a circuit block diagram illustrating another embodiment of the sound supply system and its sound adjustment device according to the disclosure. A sound supply system 60 of this embodiment is generally similar to the sound supply system 50 shown in FIG. 1, with the differences primarily described here. In this embodiment, the sound supply system 60 includes a sound adjustment device 102 that further includes an audio source 160, and a processor 150 is built into the sound adjustment device 102. The audio source 160 is signally connected to the audio signal receiving element 110 and is used to provide the audio signal. For example, the audio source 160 may have audio signals pre-stored internally. The audio signals may include general music, user-pre-recorded audio, program-generated audio, or other types of audio.

In an unillustrated embodiment, only the audio source may be built into the sound adjustment device, while the processor is not part of the sound adjustment device. In another unillustrated embodiment, only the processor may be built into the sound adjustment device, while the audio source is not part of the sound adjustment device.

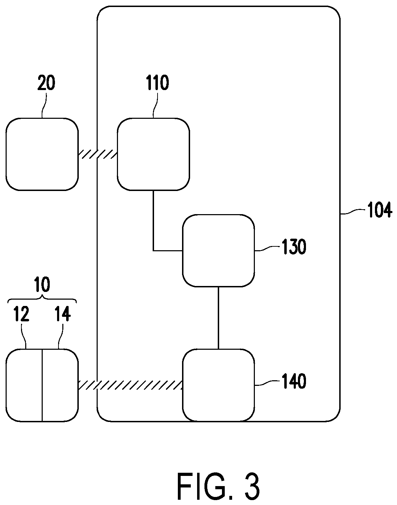

FIG. 3 is a circuit block diagram illustrating yet another embodiment of the sound adjustment device according to the disclosure. A sound adjustment device 104 of this embodiment is generally similar to the sound adjustment device 100 shown in FIG. 1, with the differences primarily described here. The sound adjustment device 104 of this embodiment includes the audio signal receiving element 110, the frequency adjuster 130, and the output port 140, but does not include the physiological state information receiving element 120 shown in FIG. 1. The frequency difference is determined by the frequency adjuster 130. Therefore, in this embodiment, the sound adjustment device 104 does not determine the frequency difference based on physiological state information. Instead, the frequency difference is determined using a preset mode or a mode chosen by the user, allowing the user to achieve corresponding effects while listening to the sound waves.

In summary, in the sound adjustment device and sound supply system of the disclosure, the sound waves received by both ears have a frequency difference, and the frequency difference is determined by the frequency adjuster. For example, but not limited to, the frequency difference may be determined based on physiological state information. When the frequency difference is determined based on physiological state information, more precise information may be used to assess the user's physiological state, and it may be determined what frequency difference is needed by the user at that time. This allows the user to experience corresponding effects after hearing the sound waves.

## Claims

1. A sound adjustment device (100, 102, 104), comprising:
an audio signal receiving element (110), used to receive an audio signal;
a frequency adjuster (130), signally connected to the audio signal receiving element (110), and used to adjust the audio signal into a left ear audio signal and a right ear audio signal, wherein a first frequency of the left ear audio signal and a second frequency of the right ear audio signal have a frequency difference, and the frequency difference is determined by the frequency adjuster (130); and
an output port (140), connected to an earphone (10), and used to output the left ear audio signal to a left ear speaker (12) of the earphone (10) and output the right ear audio signal to a right ear speaker (14) of the earphone (10).

2. The sound adjustment device (100, 102, 104) according to claim 1, wherein the frequency difference automatically cycles and changes over a time.

3. The sound adjustment device (100, 102, 104) according to claim 1, wherein the output port (140) is a wired connector or a wireless signal transmitter.

4. The sound adjustment device (100, 102, 104) according to claim 1, further comprising an audio source (20, 160), signally connected to the audio signal receiving element (110) and used to provide the audio signal.

5. The sound adjustment device (100, 102, 104) according to claim 4, wherein the audio source (20, 160) stores the audio signal or generates the audio signal through a program.

6. The sound adjustment device (100, 102, 104) according to claim 1, wherein the sound adjustment device (100, 102, 104) is a mobile phone.

7. A sound supply system (50, 60), comprising:
a physiological information sensor (52), used to sense a physiological information of a user;
a processor (54), signally connected to the physiological information sensor (52) to receive the physiological information, and generating a physiological state information according to the physiological information;
a sound adjustment device (100, 102, 104), comprising:
an audio signal receiving element (110), used to receive an audio signal;
a physiological state information receiving element (120), signally connected to the processor (54) to receive the physiological state information;
a frequency adjuster (130), signally connected to the audio signal receiving element (110) and the physiological state information receiving element (120), and used to adjust the audio signal into a left ear audio signal and a right ear audio signal, wherein a first frequency of the left ear audio signal and a second frequency of the right ear audio signal have a frequency difference, and the frequency difference is determined by the frequency adjuster (130) according to the physiological state information; and
an output port (140), used to output the left ear audio signal to a left ear speaker (12) and output the right ear audio signal to a right ear speaker (14).

8. The sound supply system (50, 60) according to claim 7, wherein the frequency difference automatically cycles and changes over a time.

9. The sound supply system (50, 60) according to claim 7, wherein the physiological state information indicates whether the physiological state of the user is awake, about to fall asleep, or sleeping.

10. The sound supply system (50, 60) according to claim 7, wherein the sound adjustment device (100, 102, 104) further comprises an audio source (20, 160), signally connected to the audio signal receiving element (110) and used to provide the audio signal.

11. The sound supply system (50, 60) according to claim 10, wherein the audio source (20, 160) stores the audio signal or generates the audio signal through a program.

12. The sound supply system (50, 60) according to claim 7, wherein the processor (54) is separate from the sound adjustment device (100, 102, 104), and the physiological state information receiving element (120) wirelessly receives the physiological state information.

13. The sound supply system (50, 60) according to claim 7, wherein the processor (54) is built into the sound adjustment device (100, 102, 104).

14. The sound supply system (50, 60) according to claim 7, wherein the sound adjustment device (100, 102, 104) is a mobile phone.
